# FASCICULE DE BREVET EUROPEEN

(11) **EP 1 011 624 B1**
(45) Date de publication et mention de la délivrance du brevet: **12.11.2003**
(21) Numéro de dépôt: 99929393.9
(22) Date de dépôt: 05.07.1999
(51) Int. Cl.: A61K 7/42

(54) **COMPOSITION COSMETIQUE PHOTOPROTECTRICE CONTENANT UN TENSIO-ACTIF ANIONIQUE, DES COMPOSES FILTRANT LE RAYONNEMENT ULTRAVIOLET ET UN COMPOSE CATIONIQUE OU ZWITTERIONIQUE AMPHIPHILE ET SON UTILISATION**
EIN ANIONISCHES TENSID, UV-STRAHLUNG FILTRIERENDE VERBINDUNGEN UND EINE KATIONISCHE VERBINDUNG ENTHALTENDE KOSMETISCHE SONNENSCHUTZZUSAMMENSETZUNG
PHOTOPROTECTANT COSMETIC COMPOSITION CONTAINING AN ANIONIC SURFACTANT, COMPOSITIONS FILTERING ULTRAVIOLET RADIATION AND AN AMPHIPHILIC CATIONIC OR DIPOLAR ION COMPOUND AND ITS USE

(30) Priorité: 09.07.1998 FR 9808828
(43) Date de publication de la demande: 28.06.2000
(73) Titulaire: L'OREAL, 75008 Paris (FR)
(72) Inventeur: ALLARD, Delphine, F-92700 Colombes (FR); CANDAU, Didier, F-91570 Bièvres (FR); MORGANTINI, Luc, F-60810 Rully (FR)
(74) Mandataire: Casalonga, Axel
(86) Numéro de dépôt international: FR9901608
(87) Numéro de publication internationale: WO00002529

(56) Documents cités:
- EP-A- 0 386 898
- EP-A- 0 603 080
- WO-A-97/28785
- US-A- 4 970 216
- US-A- 5 045 307
- US-A- 5 348 736
- DATABASE CHEMICAL ABSTRACTS [Online] STN abrégé 121:65 297, XP002102399 & JP 06 072830 A (LION CORP) 15 mars 1994 (1994-03-15)

## Description

La présente invention a pour objet une composition cosmétique à usage topique plus particulièrement destinée à la photoprotection de la peau et/ou des cheveux contre le rayonnement ultraviolet, ainsi que son utilisation dans l'application cosmétique susmentionnée. Plus particulièrement, la présente invention concerne une composition photoprotectrice sous forme de dispersion comprenant deux phases non miscibles stabilisées par au moins un tensio-actif anionique, des composés filtrant le rayonnement UV et un composé cationique ou zwitterionique amphiphile.

On sait que les radiations lumineuses de longueurs d'onde comprises entre 280 nm et 400 nm permettent le brunissement de l'épiderme humain et que les rayons de longueurs d'onde comprises entre 280 nm et 320 nm, connues sous la dénomination d'UV-B, provoquent des érythèmes et des brûlures cutanées qui peuvent nuire au développement du bronzage naturel ; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photoallergiques. Il est donc souhaitable de filtrer aussi le rayonnement UV-A.

De nombreuses compositions cosmétiques destinées à la photoprotection (UV-A et/ou UV-B) de la peau ont été proposées à ce jour.

On connaît dans l'état de la technique des compositions à base de composés actifs comportant une fonction acide, libre ou au moins partiellement neutralisée, filtrant les rayons UV. On connaît en particulier certains composés sulfoniques pour leurs bonnes propriétés filtrantes des radiations lumineuses de longueurs d'onde comprises entre 280 et 400 nm et plus particulièrement entre 280 et 360 nm, leur stabilité thermique et leur stabilité photochimique.

Cependant, la formulation de produits à indice de protection (IP) très élevé implique l'utilisation de fortes concentrations en filtres UV, ce qui n'est pas souhaitable, ni d'un point de vue toxicologique dans certains cas, ni d'un point de vue économique. L'indice de protection (IP) s'exprime mathématiquement par le rapport du temps d'irradiation nécessaire pour atteindre le seuil érythématogène avec les filtres UV au temps nécessaire pour atteindre le seuil érythématogène sans filtres UV.

Il est donc important de pouvoir proposer de nouveaux systèmes de formulation permettant d'améliorer l'indice de protection pour une même teneur en filtres et par conséquent, de pouvoir réduire les teneurs en filtres UV dans les formulations.

A la suite d'importantes recherches menées dans ce domaine, la demanderesse a découvert, de façon inattendue et surprenante, qu'il était possible de résoudre ce problème et d'améliorer l'indice de protection des filtres en modifiant le comportement interfacial du tensio-actif anionique par utilisation d'un composé cationique ou zwitterionique amphiphile qui conduit avec le tensio-actif anionique à la formation d'un composé capable d'abaisser la tension interfaciale eau/huile de paraffine à 40°C de plus de 14 mN.m⁻¹ pour une concentration de tensio-actif anionique de 0,1mmole/100 g, de plus de 26 mN.m⁻¹ pour une concentration de tensio-actif anionique de 0,5 mmole/100 g et de plus de 33 mN.m⁻¹ pour une concentration de tensio-actif anionique de 1 mmole/100 g.

Cette découverte est à la base de la présente invention.

La présente invention a donc pour objet une composition cosmétique à usage topique, en particulier pour la photoprotection de la peau et/ou des cheveux, sous forme de dispersion comprenant deux phases non miscibles stabilisées par au moins un tensio-actif anionique choisi parmi les sels d'acides gras et de métaux mono- ou polyvalents, d'ammonium ou de bases organiques, un composé filtrant le rayonnement ultraviolet constitué par un filtre hydrophile comprenant au moins une fonction acide sulfonique partiellement ou totalement neutralisée, qui est susceptible d'être adsorbé à l'interface desdites phases non miscibles, ayant pour formule 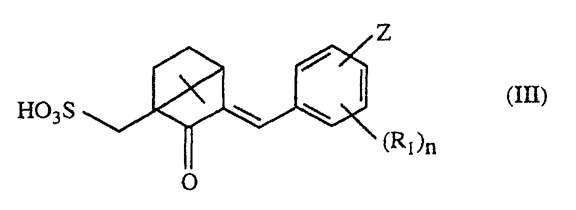 dans laquelle :
- Z désigne un groupement de formule : 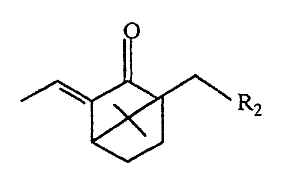
- R₂ désignant -H ou -SO₃H ;
- n désigne 0 ou un nombre entier supérieur ou égal à 1 et inférieur ou égal à 4 ;
- R₁ représente un ou plusieurs radicaux alkyle ou alcoxy, identiques ou différents, linéaires ou ramifiés, contenant de 1 à 4 atomes de carbone,
les deux radicaux méthylidène camphre se trouvant sur le noyau phényle en position méta ou para l'un par rapport à l'autre,
ainsi qu'un nanopigment d'oxyde métallique choisi parmi les oxydes de titane, de zinc, de fer, de zirconium, de cérium, et leurs mélanges, enrobés avec des agents d'enrobage hydrophobes hydrocarbonés, ayant une taille moyenne de particules primaires allant de 5 à 100 nm, composition caractérisée par le fait qu'elle contient en outre un composé cationique ou zwitterionique amphiphile qui conduit avec le tensio-actif anionique à la formation d'un composé capable d'abaisser la tension interfaciale eau-huile de paraffine à 40°C de plus de 14 mN.m⁻¹ pour une concentration de tensio-actif anionique de 0,1 mmole/100 g, de plus de 26 mN.m⁻¹ pour une concentration de tensio-actif anionique de 0,5 mmole/100 g et de plus de 33 mN.m⁻¹ pour une concentration de tensio-actif anionique de 1 mmole/100 g.

La tension interfaciale est mesurée selon la méthode de l'anneau de Lecomte du Noüy décrite par exemple dans Galenica, (F. Puisieux, M. Seiller), 5 Les Systèmes Dispersés, I Agents de surface et Emulsions, chapitre 2, p. 86-89.

Le composé cationique ou zwitterionique amphiphile peut être choisi parmi les composés ayant pour formule: 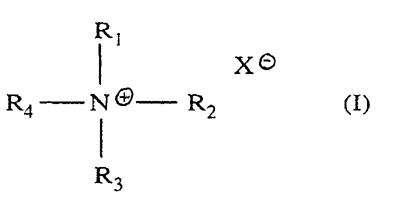 dans laquelle:
- R₁ à R₄, identiques ou différents, représentent un radical alkyle, hydroxyalkyle, alkylcarboxylate, alkylsulfonate, hydroxyalkylsulfonate, cycloalkyle, alcényle, aryle ou arylalkyle en C₁-C₃₀, linéaire ou ramifié, pouvant être interrompu par un ou plusieurs hétéroatomes tels que l'oxygène ou le soufre ou par un groupe -NH- ou amide -CONH-, ou R₁ et R₂ et/ou R₃ et R₄ forment ensemble avec l'atome d'azote auquel ils sont liés, un hétérocycle à cinq ou six chaînons, saturé ou insaturé, non substitué ou substitué par des radicaux alkyle, hydroxyalkyle, alcényle, aryle ou arylalkyle en C₁-C₃₀, au moins l'un des radicaux R₁ à R₄ ou des substituants de l'hétérocycle et de préférence au plus deux de ces radicaux ou substituants comportant au moins 6 atomes de carbone ;
- X^{⊖} représente un ion minéral ou organique choisi parmi les ions halogénure, sulfate, bisulfate, méthosulfate, paratoluènesulfonate, saecharinate, de préférence chlorure ou bromure ; étant entendu que lorsqu'au moins l'un des radicaux R₁ à R₄ représente un radical alkylcarboxylate, alkylsulfonate ou hydroxyalkylsulfonate, l'ion X^{⊖} n'existe pas.

Le composé cationique ou zwitterionique amphiphile peut également avoir pour formule la formule (II) suivante : 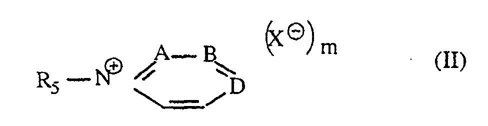 dans laquelle :
- R₅ représente un radical alkyle, hydroxyalkyle, alkylcarboxylate, cycloalkyle, alcényle, aryle, arylalkyle en C₁-C₃₀, linéaire ou ramifié, pouvant être interrompu par un ou plusieurs hétéroatomes tels que l'oxygène ou le soufre ou par un groupe -NH- ou amide -CONH- ;
- A, B ou D représentent CH, N, N^{⊕}R₆, R₆ représentant un atome d'hydrogène, un radical alkyle en C₁-C₃₀ substitué ou non substitué, linéaire ou ramifié, saturé ou insaturé, pouvant être interrompu par un ou plusieurs hétéroatomes, étant entendu qu'un seul des symboles A, B ou D représente N ou N^{⊕}R₆ ;
   sous réserve que l'un au moins des radicaux R₅ ou R₆ comporte au moins 6 atomes de carbone ;
- X^{⊖} représente un ion minéral ou organique choisi parmi les ions halogénure, sulfate, bisulfate, méthosulfate, paratoluènesulfonate, saccharinate, de préférence chlorure ou bromure ;
- m = 0, 1 ou 2.

Les radicaux R₁ à R₆ comportant au moins 6 atomes de carbone sont de préférence des radicaux en C₈-C₂₂.

Le composé cationique ou zwitterionique amphiphile de formule (I) est choisi de préférence parmi les bétaïnes telles que la décyl bétaïne, la lauryl bétaïne, la lauramidopropyl bétaïne, la myristyl bétaïne, la myristamidopropyl bétaïne, la coco-bétaïne, la cocamidoéthyl bétaïne, la cocoamido-propyl bétaïne, la cétyl bétaïne, la palmamido-propyl bétaïne, la palmitamidopropyl bétaïne, la ricinoléamido-propyl bétaïne, la stéaramidopropyl bétaïne, la stéaryl bétaïne, l'oléyl bétaïne, l'oléamidopropyl bétaïne, la béhényl bétaïne, les sultaïnes telles que la lauryl sultaïne, la lauryl hydroxysultaïne, la coco-sultaïne, la coco-hydroxysultaïne, la cocamidopropyl hydroxysultaïne, l'oléamidopropyl hydroxysultaïne, les sels d'alkyltriméthylammonium tels que le bromure ou le chlorure de dodécyltriméthylammonium, le chlorure de cocotriméthylammonium, le chlorure, bromure, méthosulfate ou tosylate de cétyltriméthylammonium, le chlorure de suif (hydrogéné) triméthylammonium, le chlorure de stéaryltriméthylammonium, le chlorure d'octyldodécyltriméthyl-ammonium, le chlorure ou méthosulfate de béhényltriméthyl-ammonium, ou encore le chlorure, le bromure ou le saccharinate de benzalkonium, le chlorure de cétalkonium, le bromure de cétéaralkonium, le chlorure ou bromure de lauralkonium, le chlorure de stéaralkonium, le chlorure d'oléalkonium, le chlorure de béhénalkonium et le chlorure de cocoyl benzyl hydroxyéthyl imidazolinium.

Le composé cationique ou zwitterionique amphiphile de formule (II) est choisi de préférence parmi le chlorure de cétylpyridinium ou le chlorure de laurylpyridinium.

A titre de composé cationique ou zwitterionique amphiphile utilisé selon l'invention, on peut citer plus particulièrement les bétaïnes.

Le composé cationique ou zwitterionique amphiphile est utilisé dans des concentrations allant de 0,1 à 15% en poids de matière active, et de préférence de 0,1 à 10% en poids de matière active, par rapport au poids total de la composition.

Un composé de formule (III) particulièrement préféré est celui correspondant à n = 0 : l'acide benzène-1,4-[di(3-méthylidènecampho-10-sulfonique)].

Pour neutraliser la fonction acide des filtres de formule (III) précités, on peut utiliser les hydroxydes de métaux mono- ou polyvalents, l'ammoniaque, ou les bases organiques telles que les alkylamines ou les alcanolamines.

Les composés filtres de formule (III) utilisés dans les compositions de l'invention sont présents dans des concentrations allant de 0,1 à 20 % en poids de matière active par rapport au poids total de la composition, plus particulièrement de 0,2 à 10 % en poids de matière active et de préférence de 0,5 à 5 % en poids de matière active.

Les nanopigments d'oxydes métalliques utilisés selon l'invention ont de préférence une taille moyenne de particules primaires allant de 10 à 50 nm.

A titre préférentiel, on utilise un nanopigment d'oxyde de titane amorphe ou cristallisé sous forme rutile et/ou anatase.

L'enrobage hydrophobe hydrocarboné du nanopigment comprend de préférence un acide gras ou un sel d'acide gras et de métal mono- ou polyvalent, d'ammonium ou de base organique.

Un nanopigment particulièrement préféré est le nanopigment d'oxyde de titane enrobé d'alumine et stéarate d'aluminium, vendu sous la dénomination "Microtitanium Dioxide MT-100 T" ou "MT-100 TV" par la Société TAYCA, ou encore le nanopigment d'oxyde de titane rutile enrobé d'acide stéarique, vendu sous la dénomination "TTO-S4" par la Société ISHIHARA SANGYO.

Le nanopigment d'oxyde métallique est présent dans la composition photoprotectrice selon l'invention à raison de 0,1 à 15% en poids et de préférence de 0,5 à 10% en poids, sur la base du poids total de la composition.

Les tensio-actifs anioniques sont présents dans des concentrations allant de préférence de 0,5 à 10 % en poids de matière active par rapport au poids total de la composition, et plus particulièrement de 0,5 à 5 % de matière active.

La composition selon l'invention peut contenir en outre au moins un tensio-actif non-ionique, choisi parmi les composés à liaison ester tels que les esters de glycols et d'acides gras, les esters du glycérol et d'acides gras, les esters de polyglycérols et d'acides gras, les esters de tétritol, pentitol et d'hexitol et d'acides gras, les esters de polyéthylèneglycols et d'acides gras, les esters de saccharose et d'acides gras, les esters de saccharose et de triglycérides, les esters de sorbitanne et d'acides gras et les esters de sorbitanne polyoxyéthylénés ou polysorbates, les composés à liaison éther tels que les éthers de polyoxyéthylèneglycols et d'alkylphénols et les éthers de polyoxyéthylèneglycols et d'alcools gras, les composés à liaison amide tels que les alkylamides polyoxyéthylénés et les copolymères d'oxydes d'alkylène.

Les tensio-actifs non-ioniques peuvent être présents dans des concentrations allant de 0,5 à 5 % en poids de matière active par rapport au poids total de la composition, et plus particulièrement de 1 à 3 % en poids de matière active.

Les compositions cosmétiques selon l'invention peuvent contenir un ou plusieurs filtres solaires complémentaires actifs dans l'UV-A et/ou l'UV-B, hydrophiles ou lipophiles autres, bien sûr, que les filtres définis ci-dessus.

Ces filtres complémentaires peuvent être notamment choisis parmi les dérivés cinnamiques, les dérivés salicyliques, les dérivés du camphre, les dérivés de triazine tels que ceux décrits dans les demandes de brevet EP-A-863145, EP-A-517104, EP-A-570838, EP-A-796851, EP-A-775698 et EP-A-878469, les dérivés de la benzophénone, les dérivés du dibenzoylméthane, les dérivés de β,β'-diphénylacrylate, les dérivés de benzimidazole, les dérivés de bis(benzotriazolylphénol) tels que décrits dans les demandes de brevet GB-A-2303549, DE-A-19726184 et EP-A-893119, les dérivés de l'acide p-aminobenzoïque, les polymères filtres et silicones filtres décrits dans la demande WO-93/04665, ainsi que les composés comportant au moins deux groupes benzoazolyle tels que décrits dans la demande de brevet EP-A-669323.

Comme exemples de filtres solaires complémentaires actifs dans l'UV-A et/ou l'UV-B, on peut citer :
l'acide p-aminobenzoïque,
le p-aminobenzoate oxyéthyléné (25 moles),
le p-diméthylaminobenzoate de 2-éthylhexyle,
le p-aminobenzoate d'éthyle N-oxypropyléné,
le p-aminobenzoate de glycérol,
le salicylate d'homomenthyle,
le salicylate de 2-éthylhexyle,
le salicylate de triéthanolamine,
le salicylate de 4-isopropylbenzyle,
le 4-tert-butyl-4'-méthoxy-dibenzoylméthane,
le 4-isopropyl-dibenzoylméthane,
le 4-méthoxy cinnamate de 2-éthylhexyle,
le diisopropyl cinnamate de méthyle,
le 4-méthoxy cinnamate d'isoamyle,
le 4-méthoxy cinnamate de diéthanolamine,
l'anthranilate de menthyle,
le 2-éthylhexyl-2-cyano-3,3'-diphénylacrylate,
l'éthyl-2-cyano-3,3'-diphénylacrylate,
l'acide 2-phénylbenzimidazole 5-sulfonique et ses sels,
le 3-(4'-triméthylammonium)-benzylidène-bornan-2-one
méthylsulfate,
la 2-hydroxy-4-méthoxybenzophénone,
le 2-hydroxy-4-méthoxybenzophénone-5-sulfonate,
la 2,4-dihydroxybenzophénone,
la 2,2',4,4'-tétrahydroxybenzophénone,
la 2,2'-dihydroxy-4,4'-diméthoxybenzophénone,
la 2-hydroxy-4-n-octoxybenzophénone,
la 2-hydroxy-4-méthoxy-4'-méthylbenzophénone,
l'acide α-(2-oxoborn-3-ylidène)-tolyl-4-sulfonique
et ses sels,
la 3-(4'-sulfo)benzylidène-bornan-2-one et ses sels,
le 3-(4'-méthylbenzylidène)-d,l-camphre,
le 3-benzylidène-d,l-camphre,
l'acide urocanique,
la 2,4,6-tris-[p-(2'-éthylhexyl-1'-oxycarbonyl)anilino]-1,3,5-triazine,
la 2-[(p-(tertiobutylamido)anilino]-4,6-bis-[(p-(2'-éthylhexyl-1'-oxycarbonyl)anilino]-1,3,5-triazine,
la 2,4-bis {[4-(2-éthylhexyloxy)-2-hydroxy]-phényl}-6-(4-méthoxy-phényl)-1,3,5-triazine,
le polymère de N-(2 et 4)-[(2-oxobom-3-ylidène)méthyl) benzyl]-acrylamide,
les polyorganosiloxanes à fonction malonate,
l'acide 1,4-bis-benzimidazolyl-phénylène-3,3',5,5'-tétrasulfonique ainsi que ses sels,
le [2,2'-méthylène-bis-[6-(2H-benzotriazol-2-yl)-4-( 1,1,3,3-tétraméthylbutyl)phénol], vendu sous la dénomination MIXXIM BB/100 par la Société FAIRMOUNT CHEMICAL.

Les compositions selon l'invention peuvent également contenir des agents de bronzage et/ou de brunissage artificiels de la peau (agents autobronzants), tels que par exemple la dihydroxyacétone (DHA).

Les compositions de l'invention peuvent comprendre en outre des adjuvants cosmétiques choisis notamment parmi les corps gras, les solvants organiques, les épaississants et/ou les gélifiants ioniques ou non-ioniques, les adoucissants, les antioxydants, les opacifiants, les stabilisants, les émollients, les hydroxyacides, les vitamines, les actifs hydrophiles ou lipophiles comme les céramides, les réducteurs, les silicones, les agents anti-radicaux libres, les agents anti-mousse, les agents hydratants, les parfums, les conservateurs, les charges, les séquestrants, les polymères, les propulseurs, les agents alcalinisants ou acidifiants, les colorants, ou tout autre ingrédient habituellement utilisé en cosmétique.

Les corps gras peuvent être constitués par une huile ou une cire ou leur mélange, la vaseline, la paraffine, la lanoline, la lanoline hydrogénée, la lanoline acétylée ; ils comprennent également les acides gras, les alcools gras tels que l'alcool laurique, cétylique, myristique, stéarique, palmitique, oléique ainsi que le 2-octyldodécanol, les esters d'acides gras tels que le monostéarate de glycérol, le monostéarate de polyéthylèneglycol, le myristate d'isopropyle, l'adipate d'isopropyle, le palmitate d'isopropyle, le palmitate d'octyle, les benzoates d'alcools gras en C₁₂-C₁₅ (Finsolv TN de FINETEX), l'alcool myristique polyoxypropyléné à 3 moles d'oxyde de propylène (WITCONOL APM de WITCO), les triglycérides d'acides gras en C₆-C₁₈ tels que les triglycérides d'acide caprylique/caprique.

Les huiles sont choisies parmi les huiles animales, végétales, minérales ou de synthèse et notamment l'huile de palme hydrogénée, l'huile de ricin hydrogénée, l'huile de vaseline, l'huile de paraffine, l'huile de Purcellin (octanoate de stéaryle), les huiles de silicone et les isoparaffines.

Les cires sont choisies parmi les cires animales, fossiles, végétales, minérales ou de synthèse. On peut citer notamment les cires d'abeille, les cires de Carnauba, de Candelila, de canne à sucre, du Japon, les ozokérites, la cire de Montan, les cires microcristallines, les paraffines, les cires et résines de silicone.

Parmi les solvants organiques, on peut citer les alcools et polyols inférieurs tels que l'éthanol, l'isopropanol, le propylèneglycol, la glycérine et le sorbitol.

Les épaississants et/ou gélifiants peuvent être choisis parmi les acides polyacryliques réticulés, les gommes de guar et celluloses modifiées ou non telles que la gomme de guar hydroxypropylée, la méthylhydroxyéthylcellulose, la cétylhydroxyéthylcellulose, l'hydroxypropylméthylcellulose, ou l'hydroxyéthylcellulose, les silices comme par exemple la Bentone Gel MiO vendue par la société NL INDUSTRIES ou la Veegum Ultra, vendue par la société POLYPLASTIC.

Bien entendu, l'homme du métier veillera à choisir ce ou ces éventuels composés complémentaires et/ou leurs quantités de manière telle que les propriétés avantageuses attachées intrinsèquement à la composition photoprotectrice conforme à l'invention, en particulier l'indice de protection, ne soient pas, ou substantiellement pas, altérées par la ou les adjonctions envisagées.

La composition cosmétique de l'invention peut être utilisée comme composition protectrice de l'épiderme humain ou des cheveux contre les rayons ultraviolets, comme composition antisolaire ou comme produit de maquillage.

Cette composition peut se présenter en particulier sous forme d'émulsion simple ou complexe (H/E, E/H, H/E/H ou E/H/E), telle qu'une crème, un lait ou un gel-crème, de poudre, de bâtonnet solide et éventuellement être conditionnée en aérosol et se présenter sous forme de mousse ou de spray.

Les compositions de l'invention peuvent être préparées selon les techniques bien connues de l'homme du métier, en particulier celles destinées à la préparation d'émulsions huile-dans-eau ou eau-dans-huile.

Lorsqu'il s'agit d'une émulsion, la phase aqueuse de celle-ci peut comprendre une dispersion vésiculaire non-ionique préparée selon des procédés connus (Bangham, Standish and Watkins, J. Mol. Biol. 13, 238 (1965), FR-A-2 315 991 et FR-A-2 416 008).

Un autre objet de la présente invention est un procédé de traitement non-thérapeutique de la peau ou des cheveux destiné à les protéger contre les effets des rayons UV consistant à appliquer sur ceux-ci une quantité efficace d'une composition cosmétique telle que définie ci-dessus.

Lorsque la composition cosmétique selon l'invention est utilisée pour la protection de l'épiderme humain contre les rayons UV, ou comme composition antisolaire, elle peut se présenter sous forme de dispersion dans des solvants ou des corps gras, sous forme de dispersion vésiculaire non-ionique ou encore sous forme d'émulsion telle qu'une crème, un lait ou un gel-crème, sous forme de bâtonnet solide et être conditionnée en aérosol et se présenter sous forme de mousse ou de spray.

Lorsque la composition cosmétique selon l'invention est utilisée pour la protection des cheveux, elle peut se présenter sous forme de shampooing, d'émulsion, de dispersion vésiculaire non-ionique, être conditionnée en aérosol et se présenter sous forme de mousse ou spray ; elle peut constituer par exemple une composition à rincer, à appliquer avant ou après shampooing, avant ou après coloration ou décoloration, avant, pendant ou après permanente ou défrisage, une composition de permanente ou de défrisage, de coloration ou décoloration des cheveux.

Lorsque la composition est utilisée comme produit de maquillage des cils, des sourcils ou de la peau, tel que crème de traitement de l'édiperme, fond de teint, bâton de rouge à lèvres, fard à paupières, fard à joues, mascara ou ligneur encore appelé "eye liner", elle peut se présenter sous forme liquide, solide ou pâteuse, anhydre ou aqueuse, comme des émulsions simples ou multiples ou encore des dispersions vésiculaires non-ioniques, des poudres ou des bâtonnets solides.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95 % en poids, de préférence de 70 à 90% en poids, par rapport à l'ensemble de la formulation et la phase huileuse (comprenant notamment les filtres lipophiles) de 5 à 50% en poids, de préférence de 10 à 30 % en poids, par rapport à l'ensemble de la formulation.

L'invention sera mieux illustrée par les exemples ci-après.

### EXEMPLE 1

On a préparé une composition antisolaire conforme à l'invention se présentant sous la forme d'une émulsion de type huile-dans-eau, contenant les constituants suivants dont les proportions sont exprimées en % en poids par rapport au poids total de la composition :
- Mélange de stéarate de glycérol et de stéarate de polyéthylèneglycol à 100 moles d'oxyde d'éthylène vendu sous la dénomination "ARLACEL 165" par la société ICI 2%
- Acide stéarique 2,5%
- Alcool cétylique 0,5%
- Gomme de silicone : polydiméthylsiloxane α, ω-dihydroxylé dans cyclométhicone vendue sous la dénomination DC-2-9071 par la société DOW CORNING 5,5%
- Triglycérides d'acides gras 4%
- Isoparaffine 3%
- Beurre de karité 1,5%
- Huile de jojoba 1,5%
- Nanopigment d'oxyde de titane vendu sous la dénomination "MICROTITANIUM DIOXIDE MT-100T" par la société TAYCA 5%
- 2-cyano-3,3-diphénylacrylate de 2-éthylhexyle vendu sous la dénomination "UVINUL N-539" par la société BASF 10%
- 4 tert.butyl-4'-méthoxy dibenzoylméthane vendu sous la dénomination "PARSOL 1789" par la société ROCHE 2%
- Glycérine 4%
- Propylèneglycol 4%
- Acide benzène 1,4-[di(3-méthylidènecampho-10-sulfonique)] 0,5% MA
- Coco-bétaïne 2% MA
- Copolymère acide acrylique/acrylate d'alkyle (C₁₀-C₃₀) réticulé vendu sous la dénomination "PEMULEN TR1" par la société GOODRICH 0,12%
- Hydroxypropylméthylcellulose 0,1%
- Triéthanolamine 0,83%
- Conservateurs qs
- Parfum qs
- Eau déminéralisée qsp 100 %

On a également préparé les compositions comparatives A et B de même composition que celle ci-dessus, la composition A contenant les filtres UV liposolubles, le filtre à fonction acide et le nanopigment, mais ne contenant pas le composé zwitterionique amphiphile coco-bétaïne et la composition B contenant uniquement le composé zwitterionique amphiphile coco-bétaïne, mais ne contenant pas de filtres ni de nanopigment.

On a réalisé chacune de ces émulsions en dissolvant les filtres UV liposolubles dans la phase grasse, puis en ajoutant les émulsionnants dans cette phase grasse portée aux environs de 80°C, et enfin en additionnant sous agitation rapide, la phase aqueuse contenant l'acide benzène 1,4-[di(3-méthylidènecampho-10-sulfonique)], neutralisé par la triéthanolamine, la phase aqueuse étant préalablement chauffée à cette même température.

### EXEMPLE 2

On a préparé une composition antisolaire conforme à l'invention en remplaçant la coco-bétaïne par la lauramidopropyl bétaïne utilisée à la même concentration.

### EXEMPLE 3

On a remplacé les bétaïnes des exemples 1 et 2 par le chlorure de dodécyltriméthylammonium, utilisé à la même concentration.

Pour chacune des formulations ainsi préparées, on a ensuite déterminé l'indice de protection solaire qui leur est attaché. Celui-ci a été déterminé en utilisant la méthode *in vitro* décrite par B.L. DIFFEY et al. dans J. Soc. Cosmet. Chem. 40-127-133 (1989) ; cette méthode consiste à déterminer les indices de protection monochromatiques tous les 5 nm dans une gamme de longueurs d'onde de 290 à 400 nm et à calculer à partir de ceux-ci l'indice de protection selon une équation mathématique donnée.

L'indice de protection moyen calculé à partir de trois valeurs mesurées et l'écart-type sont indiqués dans le tableau ci-dessous.

| | Exemple 1 | Exemple comparatif A | Exemple comparatif B | Exemple 2 | Exemple 3 |
|---|---|---|---|---|---|
| IP moyen | 31,9 | 16,8 | 1 | 33 | 27,7 |
| (écart-type) | (6,6) | (0,1) | | (3) | (7,4) |

Ces résultats démontrent clairement que pour le même système photoprotecteur, à savoir nanopigment + filtres UV liposolubles + acide benzène 1,4-[di(3-méthylidènecampho-10-sulfonique)], la composition de l'exemple 1 selon l'invention contenant le composé amphiphile zwitterionique coco-bétaïne présente un indice de protection significativement plus élevé que celle de l'exemple comparatif A, alors que le composé amphiphile zwitterionique par lui-même ne présente pas de pouvoir photoprotecteur (voir exemple comparatif B).

Ces résultats ont été confirmés par la méthode in *vivo* qui consiste à appliquer les formulations, à raison de 2 mg de produit/cm² de peau, sur le dos de 5 sujets humains, puis à soumettre simultanément les zones protégées et les zones non protégées de peau à l'action d'un simulateur solaire XENON MULTIPORT WG 320-1 mm + UG 11-1mm.

Les résultats sont les suivants :

| | Exemple comparatif A | Exemple 1 | Exemple 2 | Exemple 3 |
|---|---|---|---|---|
| IP moyen | 22,5 | 41,4 | 32,2 | 28,7 |
| (écart-type) | (5,5) | (12,1) | (3,3) | (5) |

## Revendications

1. Composition cosmétique à usage topique, en particulier pour la photoprotection de la peau et/ou des cheveux, sous forme de dispersion comprenant deux phases non miscibles stabilisées par au moins un tensio-actif anionique choisi parmi les sels d'acides gras et de métaux mono- ou polyvalents, d'ammonium ou de bases organiques, un composé filtrant le rayonnement ultraviolet constitué par un filtre hydrophile comprenant au moins une fonction acide sulfonique partiellement ou totalement neutralisée, qui est susceptible d'être adsorbé à l'interface desdites phases non miscibles, ayant pour formule : 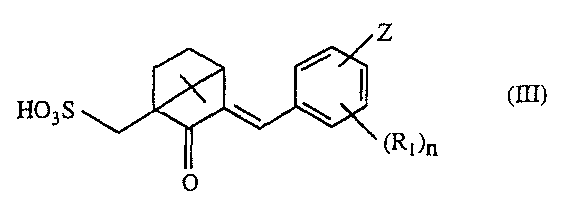 dans laquelle :
- Z désigne un groupement de formule : 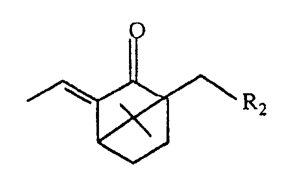
- R₂ désignant -H ou -SO₃H ;
- n désigne O ou un nombre entier supérieur ou égal à 1 et inférieur ou égal à 4 ;
- R₁ représente un ou plusieurs radicaux alkyle ou alcoxy, identiques ou différents, linéaires ou ramifiés, contenant de 1 à 4 atomes de carbone,
les deux radicaux méthylidène camphre se trouvant sur le noyau phényle en position méta ou para l'un par rapport à l'autre,
ainsi qu'un nanopigment d'oxyde métallique choisi parmi les oxydes de titane, de zinc, de fer, de zirconium, de cérium, et leurs mélanges, enrobés avec des agents d'enrobage hydrophobes hydrocarbonés, ayant une taille moyenne de particules primaires allant de 5 à 100 nm,
composition **caractérisée par le fait qu'**elle contient en outre un composé cationique ou zwitterionique amphiphile qui conduit avec le tensio-actif anionique à la formation d'un composé capable d'abaisser la tension interfaciale eau/huile de paraffine à 40°C de plus de 14 mN.m⁻¹ pour une concentration de tensio-actif anionique de 0,1 mmole/100 g, de plus de 26 mN.m⁻¹ pour une concentration de tensio-actif anionique de 0,5 mmole/100 g et de plus de 33 mN.m⁻¹ pour une concentration de tensio-actif anionique de 1 mmole/100 g.

2. Composition selon la revendication 1, **caractérisée par le fait que** le composé cationique ou zwitterionique amphiphile a pour formule : 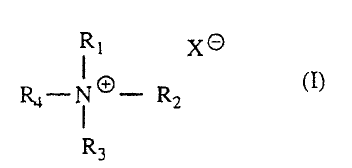 dans laquelle:
- R₁ à R₄, identiques ou différents, représentent un radical alkyle, hydroxyalkyle, alkylcarboxylate, alkylsulfonate, hydroxyalkylsulfonate, cycloalkyle, alcényle, aryle ou arylalkyle en C₁-C₃₀, linéaire ou ramifié, pouvant être interrompu par un ou plusieurs hétéroatomes tels que l'oxygène ou le soufre ou par un groupe -NH- ou amide -CONH-, ou R₁ et R₂ et/ou R₃ et R₄ forment ensemble avec l'atome d'azote auquel ils sont liés, un hétérocycle à cinq ou six chaînons, saturé ou insaturé, non substitué ou substitué par des radicaux alkyle, hydroxyalkyle, alcényle, aryle ou arylalkyle en C₁-C₃₀, au moins l'un des radicaux R₁ à R₄ ou des substituants de l'hétérocycle et de préférence au plus deux de ces radicaux ou substituants comportant au moins 6 atomes de carbone ;
- X^{⊖} représente un ion minéral ou organique choisi parmi les ions halogénure, sulfate, bisulfate, méthosulfate, paratoluènesulfonate, saccharinate, de préférence chlorure ou bromure ; étant entendu que lorsqu'au moins l'un des radicaux R₁ à R₄ représente un radical alkylcarboxylate, alkylsulfonate ou hydroxyalkylsulfonate, l'ion X^{⊖} n'existe pas.

3. Composition selon la revendication 1, **caractérisée par le fait que** le composé cationique ou zwitterionique amphiphile a pour formule : 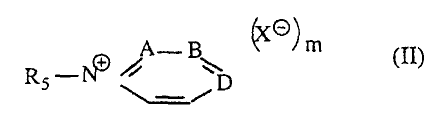 dans laquelle :
- R₅ représente un radical alkyle, hydroxyalkyle, alkylcarboxylate, cycloalkyle, alcényle, aryle, arylalkyle en C₁-C₃₀, linéaire ou ramifié, pouvant être interrompu par un ou plusieurs hétéroatomes tels que l'oxygène ou le soufre ou par un groupe -NH- ou amide -CONH- ;
- A, B ou D représentent CH, N, N^{⊕}R₆, R₆ représentant un atome d'hydrogène, un radical alkyle en C₁-C₃₀ substitué ou non substitué, linéaire ou ramifié, saturé ou insaturé, pouvant être interrompu par un ou plusieurs hétéroatomes, étant entendu qu'un seul des symboles A, B ou D représente N ou N^{⊕}R₆ ;
sous réserve que l'un au moins des radicaux R₅ ou R₆ comporte au moins 6 atomes de carbone ;
- X^{⊖} représente un ion minéral ou organique choisi parmi les ions halogénure, sulfate, bisulfate, méthosulfate, paratoluènesulfonate, saccharinate, de préférence chlorure ou bromure ;
- m = 0, 1 ou 2 ;

4. Composition selon la revendication 2 ou 3, **caractérisée par le fait que** les radicaux R₁ à R₆ comportant au oins 6 atomes de carbone sont des radicaux en C₈-C₂₂.

5. Composition selon la revendication 2 ou 4, **caractérisée par le fait que** le composé cationique ou zwitterionique amphiphile de formule (I) est choisi parmi les bétaïnes telles que la décyl bétaïne, la lauryl bétaïne, la lauramidopropyl bétaïne, la myristyl bétaïne, la myristamidopropyl bétaïne, la coco-bétaïne, la cocamidoéthyl bétaïne, la cocoamido-propyl bétaïne, la cétyl bétaïne, la palmamidopropyl bétaïne, la palmitamidopropyl bétaïne, la ricinoléamidopropyl bétaïne, la stéaramidopropyl bétaïne, la stéaryl bétaïne, l'oléyl bétaïne, l'oléamidopropyl bétaïne et la béhényl bétaïne, les sultaïnes telles que la lauryl sultaïne, la lauryl hydroxysultaïne, la coco-sultaïne, la coco-hydroxysultaïne, la cocamidopropyl hydroxysultaïne et l'oléamidopropyl hydroxysultaïne, les sels d'alkyltriméthylammonium tels que le bromure ou le chlorure de dodécyltriméthylammonium, le chlorure de cocotriméthylammonium, le chlorure, bromure, méthosulfate ou tosylate de cétyltriméthylammonium, le chlorure de suif (hydrogéné) triméthylammonium, le chlorure de stéaryl-triméthylammonium, le chlorure d'octyldodécyltriméthylammonium, le chlorure ou méthosulfate de béhényltriméthylammonium, ou encore le chlorure, le bromure ou le saccharinate de benzalkonium, le chlorure de cétalkonium, le bromure de cétéaralkonium, le chlorure ou bromure de lauralkonium, le chlorure de stéaralkonium, le chlorure d'oléalkonium, le chlorure de béhénalkonium et le chlorure de cocoyl benzyl hydroxyéthyl imidazolinium.

6. Composition selon la revendication 3 ou 4, **caractérisée par le fait que** le composé cationique ou zwitterionique amphiphile dé formule (II) est choisi parmi le chlorure de cétylpyridinium et le chlorure de laurylpyridinium.

7. Composition selon l'une quelconque des revendications précédentes, **caractérisée par le fait que** le composé cationique ou zwitterionique amphiphile est présent dans des concentrations allant de 0,1 à 15% en poids de matière active et de préférence de 0,1 à 10% en poids de matière active, par rapport au poids total de la composition.

8. Composition selon l'une quelconque des revendications précédentes, **caractérisée par le fait que** le composé filtrant le rayonnement ultraviolet de formule (III) est l'acide benzène 1,4-[di(3-méthylidènecampho-10-sulfonique)].

9. Composition selon l'une quelconque des revendications précédentes, **caractérisée par le fait que** la fonction acide sulfonique du composé filtrant le rayonnement ultraviolet de formule (III) est neutralisée par un hydroxyde de métal mono- ou polyvalent, l'ammoniaque, ou une base organique.

10. Composition selon l'une quelconque des revendications précédentes, **caractérisée par le fait que** le composé filtrant de formule (III) est présent dans des concentrations allant de 0,1 à 20% en poids de matière active par rapport au poids total de la composition, plus particulièrement de 0,2 à 10% en poids de matière active, et de préférence de 0,5 à 5% en poids de matière active.

11. Composition selon l'une quelconque des revendications précédentes, **caractérisée par le fait que** le tensio-actif anionique est présent dans des concentrations allant de 0,5 à 10 % en poids de matière active par rapport au poids total de la composition, et plus particulièrement de 0,5 à 5 % en poids de matière active.

12. Composition selon l'une quelconque des revendications précédentes, **caractérisée par le fait que** le nanopigment d'oxyde métallique est un nanopigment d'oxyde de titane.

13. Composition selon l'une quelconque des revendications précédentes, **caractérisée par le fait que** l'agent d'enrobage hydrophobe hydrocarboné comprend un acide gras ou un sel d'acide gras et de métal mono- ou polyvalent, d'ammonium ou de base organique.

14. Composition selon l'une quelconque des revendications précédentes, **caractérisée par le fait que** le nanopigment d'oxyde métallique a une taille moyenne des particules primaires allant de 10 à 50 nm.

15. Composition selon l'une quelconque des revendications précédentes, **caractérisée par le fait que** le nanopigment d'oxyde métallique est présent à raison de 0,1 à 15% en poids par rapport au poids total de la composition, et de préférence à raison de 0,5 à 10% en poids.

16. Composition selon l'une quelconque des revendications précédentes, **caractérisée par le fait qu'**elle contient en outre au moins un tensio-actif non-ionique choisi parmi les composés à liaison ester tels que les esters de glycols et d'acides gras, les esters du glycérol et d'acides gras, les esters de polyglycérols et d'acides gras, les esters de tétritol, pentitol et d'hexitol et d'acides gras, les esters de polyéthylèneglycols et d'acides gras, les esters de saccharose et d'acides gras, les esters de saccharose et de triglycérides, les esters de sorbitanne et d'acides gras et les esters de sorbitarine polyoxyéthylènés ou polysorbates, les composés à liaison éther tels que les éthers de polyoxyéthylèneglycols et d'alkylphénols et les éthers de polyoxyéthylèneglycols et d'alcools gras, les composés à liaison amide tels que les alkylamides polyoxyéthylénés et les copolymères d'oxydes d'alkylène.

17. Composition selon la revendication 16, **caractérisée par le fait que** les tensio-actifs non-ioniques sont présents dans des concentrations allant de 0,5 à 5 % en poids de matière active par rapport au poids total de la composition, et plus particulièrement de 1 à 3 % en poids de matière active.

18. Composition selon l'une quelconque des revendications précédentes, **caractérisée par le fait qu'**elle contient en outre un ou plusieurs filtres solaires complémentaires UV-B et/ou UV-A hydrophiles ou lipophiles autres que les filtres définis dans l'une quelconque des revendications précédentes.

19. Composition selon la revendication 18, **caractérisée par le fait que** les filtres solaires complémentaires sont choisis parmi les cinnamates, les salicylates, les dérivés du benzylidène camphre, les dérivés de triazine, les dérivés de la benzophénone, les dérivés du dibenzoylméthane, les dérivés de β,β'-diphénylacrylate, les dérivés de l'acide p-aminobenzoïque, les dérivés de benzimidazole, les dérivés de bis(benzotriazolylphénol), les polymères filtres et les silicones filtres, ainsi que les composés comportant au moins deux groupes benzoazolyle.

20. Composition selon l'une quelconque des revendications précédentes, **caractérisée par le fait qu'**elle comprend en outre au moins un adjuvant cosmétique choisi parmi les corps gras, les solvants organiques, les épaississants et/ou les gélifiants ioniques ou non-ioniques, les adoucissants, les antioxydants, les opacifiants, les stabilisants, les émollients, les hydroxyacides, les vitamines, les actifs hydrophiles ou lipophiles comme les céramides, les réducteurs, les silicones, les agents anti-radicaux libres, les agents anti-mousse, les agents hydratants, les parfums, les conservateurs, les charges, les séquestrants, les polymères, les propulseurs, les agents alcalinisants ou acidifiants et les colorants.

21. Composition selon l'une quelconque des revendications précédentes, **caractérisée par le fait qu'**elle constitue une composition protectrice de l'épiderme humain ou une composition antisolaire et se présente sous forme de dispersion, émulsion telle que crème, lait ou gel-crème, de bâtonnet solide, mousse ou spray.

22. Composition selon l'une quelconque des revendications 1 à 20, **caractérisée par le fait qu'**elle constitue une composition de maquillage des cils, des sourcils ou de la peau et se présente sous forme solide, liquide ou pâteuse, telle qu'une émulsion, une dispersion vésiculaire non-ionique, une poudre ou un bâtonnet solide.

23. Composition selon l'une quelconque des revendications 1 à 20, utilisée pour la protection des cheveux contre les rayons ultraviolets, **caractérisée par le fait qu'**elle se présente sous forme de shampooing, d'émulsion, de dispersion vésiculaire non-ionique, de mousse ou spray.

24. Procédé de traitement non-thérapeutique de la peau et/ou des cheveux pour les protéger contre les effets des rayons UV de longueurs d'onde comprises entre 280 et 400 nm, **caractérisé par le fait qu'**il consiste à appliquer une quantité efficace d'une composition cosmétique photoprotectrice telle que définie dans l'une quelconque des revendications précédentes.

25. Utilisation pour augmenter l'indice de protection d'une composition cosmétique photoprotectrice selon l'une quelconque des revendications 1 à 23, d'un composé cationique ou zwitterionique amphiphile qui conduit avec le tensio-actif anionique à la formation d'un composé capable d'abaisser la tension interfaciale eau/huile de paraffine à 40°C de plus de 14 mN.m⁻¹ pour une concentration de tensio-actif anionique de 0,1 mmole/100 g, de plus de 26 mN.m⁻¹ pour une concentration de tensio-actif anionique de 0,5 mmole/100 g et de plus de 33 mN.m⁻¹ pour une concentration de tensio-actif anionique de 1 mmole/100 g.

26. Utilisation selon la revendication 25, **caractérisée par le fait que** le composé filtrant le rayonnement ultraviolet de formule (III) est l'acide benzène-1,4-[di(3-méthylidènecampho-10-sulfonique)].

27. Utilisation selon la revendication 25 ou 26, **caractérisée par le fait que** le composé cationique ou zwitterionique amphiphile est tel que défini dans l'une quelconque des revendications 2 à 6.

## Patentansprüche

1. Kosmetische Zusammensetzung für die topische Anwendung, insbesondere für den Lichtschutz der Haut und/oder der Haare, in Form einer Dispersion, die enthält: zwei nicht mischbare Phasen, die durch mindestens einen anionischen grenzflächenaktiven Stoff stabilisiert sind, der unter den Salzen aus Fettsäuren und ein- oder mehrwertigen Metallen, den Ammoniumsalzen von Fettsäuren und den Salzen aus Fettsäuren und organischen Basen ausgewählt ist, eine Verbindung, die Ultraviolett-Strahlung filtert, die aus einem hydrophilen Filter besteht, der mindestens eine Sulfonsäuregruppe enthält, die ganz oder teilweise neutralisiert ist, die imstande ist, an der Grenzfläche der nicht miteinander mischbaren Phasen adsorbiert zu werden, die der folgenden Formel 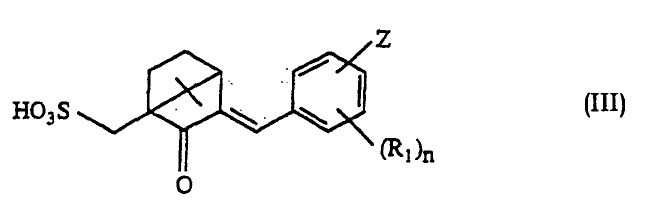 entspricht, in der bedeuten:
- Z eine Gruppe der Formel: 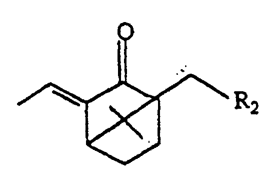 worin R₂ Wasserstoff oder -SO₃H bedeutet;
- n O oder eine ganze Zahl, die größer als oder gleich 1 und kleiner als oder gleich 4 ist;
- R₁ einen oder mehrere Alkyl- oder Alkoxyreste, die gleich oder verschieden, geradkettig oder verzweigt sind, die 1 bis 4 Kohlenstoffatome enthalten,
wobei die beiden Methylidencampherreste am Phenylring in m- oder p-Stellung, bezogen aufeinander, angeordnet sind,
sowie ein Nanopigment eines Metalloxids, das unter den Oxiden von Titan, Zink, Eisen, Zirconium, Cer und deren Gemischen ausgewählt ist, die mit hydrophoben kohlenwasserstoffhaltigen Umhüllungsmitteln umhüllt sind, das eine mittlere Größe der Primärpartikel im Bereich von 5 bis 100 nm aufweist,
wobei die Zusammensetzung **dadurch gekennzeichnet ist, dass** sie außerdem eine amphiphile zwitterionische oder kationische Verbindung enthält, die mit dem anionischen grenzflächenaktiven Stoff zur Bildung einer Verbindung führt, die imstande ist, die Grenzflächenspannung an einer Wasser/Paraffinöl-Grenzfläche bei 40 °C bei einer Konzentration an anionischem grenzflächenaktiven Stoff von 0,1 mmol/100 g um mehr als 14 mN·m⁻¹, bei einer Konzentration an anionischem grenzflächenaktiven Stoff von 0,5 mmol/100 g um mehr als 26 mN·m⁻¹ und bei einer Konzentration an anionischem grenzflächenaktiven Stoff von 1 mmol/100 g um mehr als 33 mN·m⁻¹ zu senken.

2. Zusammensetzung nach Anspruch 1, **dadurch gekennzeichnet, dass** die amphiphile zwitterionische oder kationische Verbindung der Formel 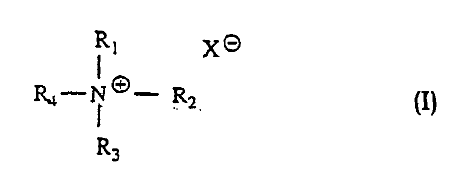 entspricht, in der bedeuten:
- R₁ bis R₄, gleich oder verschieden, Alkyl, Hydroxyalkyl, Alkylcarboxylat, Alkylsulfonat, Hydroxyalkylsulfonat, Cycloalkyl, Alkenyl, Aryl oder Arylalkyl, wobei diese Reste 1 bis 30 Kohlenstoffatome aufweisen, geradkettig oder verzweigt sind und durch ein oder mehrere Heteroatome, wie Sauerstoff oder Schwefel, oder durch eine Gruppe -NH- oder eine Amidgruppe -CONH- unterbrochen sein können, oder R₁ und R₂ und/oder R₃ und R₄ Reste, die zusammen mit dem Stickstoffatom, mit dem sie verbunden sind, einen Heterocyclus aus fünf oder sechs Kettengliedern bilden, der gesättigt oder ungesättigt, nicht substituiert oder mit Alkyl-, Hydroxyalkyl-, Alkenyl-, Aryl- oder Arylalkylresten substituiert ist, die 1 bis 30 Kohlenstoffatome aufweisen, wobei mindestens einer der Reste R₁ bis R₄ oder der Substituenten des Heterocyclus und vorzugsweise höchstens zwei dieser Reste oder Substituenten mindestens 6 Kohlenstoffatome aufweist aufweisen;
- X⁻ ein anorganisches oder organisches Ion, das unter den Halogenid-, Sulfat-, Hydrogensulfat-, Methansulfat-, p-Toluolsulfonat-, Saccharinat-Ionen, vorzugsweise Chlorid oder Bromid, ausgewählt ist, mit der Maßgabe, dass es das Ion X⁻ nicht gibt, wenn mindestens einer der Reste R₁ bis R₄ Alkylcarboxylat, Alkylsulfonat oder Hydroxyalkylsulfonat bedeutet.

3. Zusammensetzung nach Anspruch 1, **dadurch gekennzeichnet, dass** die amphiphile zwitterionische oder kationische Verbindung der Formel 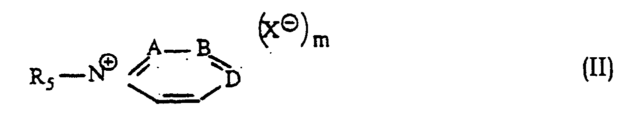 entspricht, in der bedeuten:
- R₅ einen geradkettigen oder verzweigten Alkyl-, Hydroxyalkyl-, Alkylcarboxylat-, Cycloalkyl-, Alkenyl-, Aryl-, Arylalkylrest mit 1 bis 30 Kohlenstoffatomen, der durch ein oder mehrere Heteratome, wie Sauerstoff oder Schwefel, oder eine Gruppe -NH- oder eine Amidgruppe -CONH- unterbrochen sein kann;
- A, B oder D eine Gruppe CH, N, N⁺R₆, worin R₆ Wasserstoff, substituiertes oder nicht substituiertes, geradkettiges oder verzweigtes, gesättigtes oder ungesättigtes C₁₋₃₀-Alkyl darstellt, das durch ein oder mehrere Heteroatome unterbrochen sein kann, mit der Maßgabe, dass eines der Symbole A, B oder D einem Rest N oder N⁺R₆ entspricht;
mit der Maßgabe, dass mindestens einer der Reste R₅ und R₆ mindestens 6 Kohlenstoffatome aufweist;
- X⁻ ein anorganisches oder organisches Ion, das unter den Halogenid-, Sulfat-, Hydrogensulfat-, Methansulfat-, p-Toluolsulfonat-, Saccharinat-Ionen, vorzugsweise Chlorid oder Bromid, ausgewählt ist,
- m die Zahl 0, 1 oder 2.

4. Zusammensetzung nach Anspruch 2 oder 3, **dadurch gekennzeichnet, dass** die Reste R₁ bis R₆, die mindestens 6 Kohlenstoffatome aufweisen, C₈₋₂₂-Reste sind.

5. Zusammensetzung nach Anspruch 2 oder 4, **dadurch gekennzeichnet, dass** die amphiphile zwitterionische oder kationische Verbindung der Formel (I) ausgewählt ist unter: den Betainen, wie Decylbetain, Laurylbetrain, Lauramidopropylbetain, Myristylbetain, Myristamidopropylbetain, Cocobetain, Cocoamidoethylbetain, Cocoamidopropylbetain, Cetylbetain, Palmamidopropylbetain, Palmitamidopropylbetain, Ricinoleamidopropylbetain, Stearamidopropylbetain, Stearylbetain, Oleylbetain, Oleamidopropylbetain und Behenylbetain; den Sultainen, wie Laurylsultain, Laurylhydroxysultain, Cocosultain, Cocohydroxysultain, Cocamidopropylhydroxysultain und Oleamidopropylhydroxysultain; den Alkyltrimethylammoniumsalzen, wie Dodecyltrimethylammoniumbromid- oder chlorid, Cocotrimethylammoniumchlorid, Cetyltrimethylammoniumchlorid, -bromid, -methansulfat oder -tosylat, Trimethylammonium-(hydrierter Talg)-chlorid, Trimethylammoniumstearylchlorid, Octyldodecyltrimethylammoniumchlorid, Behenyltrimethylammoniumchlorid oder -methansulfat oder auch Benzalkoniumchlorid, -bromid oder -saccharinat, Cetalkoniumchlorid, Cetearalkoniumbromid, Lauralkoniumchlorid oder -bromid, Stearalkoniumchlorid, Olealkoniumchlorid, Behenalkoniumchlorid und Cocoylbenzylhydroxyethylimidazoliniumchlorid.

6. Zusammensetzung nach Anspruch 3 oder 4, **dadurch gekennzeichnet, dass** die amphiphile zwitterionische oder kationische Verbindung der Formel (II) unter Cetylpyridiniumchlorid und Laurylpyridiniumchlorid ausgewählt ist.

7. Zusammensetzung nach einem der vorhergehenden Ansprüche, **dadurch gekennzeichnet, dass** die amphiphile zwitterionische oder kationische Verbindung in einer Konzentration enthalten ist, die im Bereich von 0,1 bis 15 Gew.-% Wirkstoff und vorzugsweise 0,1 bis 10 Gew.-% Wirkstoff, bezogen auf das Gesamtgewicht der Zusammensetzung, liegt.

8. Zusammensetzung nach einem der vorhergehenden Ansprüche, **dadurch gekennzeichnet, dass** es sich bei der die Ultraviolett-Strahlung filternden Verbindung der Formel (III) um Benzol-1,4-[di(3-methylidencampher-10-sulfonsäure)] handelt.

9. Zusammensetzung nach einem der vorhergehenden Ansprüche, **dadurch gekennzeichnet, dass** die Sulfonsäuregruppe der die UV-Strahlung filternden Verbindung der Formel (III) mit einem Hydroxid eines ein- oder mehrwertigen Metalls, Ammoniak oder einer organischen Base neutralisiert ist.

10. Zusammensetzung nach einem der vorhergehenden Ansprüche, **dadurch gekennzeichnet, dass** die filternde Verbindung der Formel (III) in einer Konzentration enthalten ist, die im Bereich von 0,1 bis 20 Gew.-% Wirkstoff, bezogen auf das Gesamtgewicht der Zusammensetzung, vor allem im Bereich von 0,2 bis 10 Gew.-% Wirkstoff und vorzugsweise im Bereich von 0,5 bis 5 Gew.-% Wirkstoff liegt.

11. Zusammensetzung nach einem der vorhergehenden Ansprüche, **dadurch gekennzeichnet, dass** der anionische grenzflächenaktive Stoff in einer Konzentration enthalten ist, die im Bereich von 0,5 bis 10 Gew.-% Wirkstoff, bezogen auf das Gesamtgewicht der Zusammensetzung, und vor allem 0,5 bis 5 Gew.-% Wirkstoff liegt.

12. Zusammensetzung nach einem der vorhergehenden Ansprüche, **dadurch gekennzeichnet, dass** es sich bei dem Nanopigment eines Metalloxids um ein Nanopigment von Titandioxid handelt.

13. Zusammensetzung nach einem der vorhergehenden Ansprüche, **dadurch gekennzeichnet, dass** das hydrophobe kohlenwasserstoffhaltige Umhüllungsmittel eine Fettsäure oder ein Salz aus einer Fettsäure und einem ein- oder mehrwertigen Metall, ein Ammoniumsalz einer Fettsäure oder ein Salz aus einer Fettsäure und einer organischen Base umfasst.

14. Zusammensetzung nach einem der vorhergehenden Ansprüche, **dadurch gekennzeichnet, dass** das Nanopigment eines Metalloxids eine mittlere Größe der Primärpartikel im Bereich von 10 bis 50 nm aufweist.

15. Zusammensetzung nach einem der vorhergehenden Ansprüche, **dadurch gekennzeichnet, dass** das Nanopigment eines Metalloxids in einem Mengenanteil von 0,1 bis 15 Gew.-%, bezogen auf das Gesamtgewicht der Zusammensetzung, und vorzugsweise in einem Mengenanteil von 0,5 bis 10 Gew.-% enthalten ist.

16. Zusammensetzung nach einem der vorhergehenden Ansprüche, **dadurch gekennzeichnet, dass** sie außerdem mindestens einen nichtionischen grenzflächenaktiven Stoff enthält, der ausgewählt ist unter den Verbindungen mit Esterbindung, wie den Glykolfettsäureestern, den Glycerinfettsäureestern, den Polyglycerinfettsäureestern, den Estern aus Tetriten, Pentiten und Hexiten und Fettsäuren, den Estern aus Polyethylenglykolen und Fettsäuren, den Estern aus Saccharose und Fettsäuren, den Estern aus Saccharose und Triglyceriden, den Estern aus Sorbitan und Fettsäuren und den polyethoxylierten Sorbitanestern oder Polysorbaten, den Verbindungen mit E-therbindung, wie den Ethern aus Polyoxyethylenglykolen und Alkylphenolen und den Ethern aus Polyoxyethylenglykolen und Fettalkoholen, den Verbindungen mit Amidbindung, wie den polyethoxylierten Alkylamiden und den Copolymeren aus Alkylenoxiden.

17. Zusammensetzung nach Anspruch 16, **dadurch gekennzeichnet, dass** die nichtionischen grenzflächenaktiven Stoffe in Konzentrationen enthalten sind, die im Bereich von 0,5 bis 5 Gew.-% Wirkstoff, bezogen auf das Gesamtgewicht der Zusammensetzung, und vor allem 1 bis 3 Gew.-% Wirkstoff liegen.

18. Zusammensetzung nach einem der vorhergehenden Ansprüche, **dadurch gekennzeichnet, dass** sie außerdem ein oder mehrere zusätzliche hydrophile oder lipophile UV-B- und/oder UV-A-Sonnenschutzfilter enthält, die verschieden von den Filtern sind, die in einem der vorhergehenden Ansprüche definiert sind.

19. Zusammensetzung nach Anspruch 18, **dadurch gekennzeichnet, dass** die zusätzlichen Sonnenschutzfilter ausgewählt sind unter den Cinnamaten, den Salicylaten, den Benzylidencampher-Derivaten, den Triazin-Derivaten, den Benzophenon-Derivaten, den Dibenzoylmethan-Derivaten, den β,β'-Diphenylacrylat-Derivaten, den p-Aminobenzoesäure-Derivaten, den Benzimidazol-Derivaten, den Bis(benzotriazolylphenol)-Derivaten, den Polymerfiltern, den Siliconfiltern, sowie den Verbindungen, die mindestens zwei Benzoazolyl-Gruppen aufweisen.

20. Zusammensetzung nach einem der vorhergehenden Ansprüche, **dadurch gekennzeichnet, dass** sie außerdem mindestens einen kosmetischen Zusatzstoff enthält, der ausgewählt ist unter den Fettsubstanzen, den organischen Lösemitteln, den ionischen oder nichtionischen Verdickungsmitteln und/oder Gelbildnern, den reizlindernden Mitteln, den Antioxidantien, den Trübungsmitteln, den Stabilisierungsmitteln, dem Weichmachern, den Hydroxysäuren, den Vitaminen, den hydrophilen oder lipophilen Wirkstoffen, wie den Ceramiden, den Reduktionsmitteln, den Siliconen, den Mitteln gegen freie Radikale, den Antischaummitteln, den Hydratisierungsmitteln, den Parfüms, den Konservierungsmitteln, den Füllstoffen, den Maskierungsmitteln, den Polymeren, den Treibmitteln, den alkalisch machenden Mitteln oder Säuerungsmitteln und den Farbmitteln.

21. Zusammensetzung nach einem der vorhergehenden Ansprüche, **dadurch gekennzeichnet, dass** sie eine Zusammensetzung zum Schutz der menschlichen Epidermis oder eine Sonnenschutzzusammensetzung ist und dass sie in Form einer Dispersion, Emulsion, wie einer Creme, Milch oder Gelcreme, in Form eines festen Stifts, eines Schaums oder Sprays vorliegt.

22. Zusammensetzung nach einem der Ansprüche 1 bis 20, **dadurch gekennzeichnet, dass** sie eine Zusammensetzung zum Schminken der Wimpern, der Augenbrauen oder der Haut bildet und dass sie in fester, flüssiger oder pastöser Form, wie einer Emulsion, einer nichtionischen Vesikeldispersion, eines Puders oder eines festen Stifts, vorliegt.

23. Zusammensetzung nach einem der Ansprüche 1 bis 20, die für den Schutz der Haare vor Ultraviolett-Strahlung verwendet wird, **dadurch gekennzeichnet, dass** sie in Form eines Haarwaschmittels, einer Emulsion, einer Dispersion nichtionischer Vesikeln, eines Schaums oder eines Sprays vorliegt.

24. Verfahren zur nicht-therapeutischen Behandlung der Haut und/oder der Haare für den Schutz vor den Auswirkungen von UV-Strahlung mit einer Wellenlänge im Bereich von 280 bis 400 nm, **dadurch gekennzeichnet, dass** es darin besteht, eine wirksame Menge einer kosmetischen Lichtschutzzusammensetzung, die wie in einem der vorhergehenden Ansprüche definiert ist, aufzutragen.

25. Verwendung einer amphiphilen zwitterionischen oder kationischen Verbindung zur Erhöhung des Lichtschutzfaktors einer kosmetischen Lichtschutzzusammensetzung nach einem der Ansprüche 1 bis 23, wobei die Verbindung mit dem anionischen grenzflächenaktiven Stoff zur Bildung einer Verbindung führt, die imstande ist, die Grenzflächenspannung an einer Wasser/Paraffinöl-Grenzfläche bei 40 °C bei einer Konzentration an anionischem grenzflächenaktivem Stoff von 0,1 mmol/100 g um mehr als 14 mN·m⁻¹, bei einer Konzentration an anionischem grenzflächenaktivem Stoff von 0,5 mmol/100 g um mehr als 26 mN·m⁻¹ und bei einer Konzentration an anionischem grenzflächenaktivem Stoff von 1 mmol/100 g um mehr als 33 mN·m⁻¹ zu senken.

26. Verwendung nach Anspruch 25, **dadurch gekennzeichnet, dass** es sich bei der die Ultraviolett-Strahlung filternden Verbindung der Formel (III) um Benzol-1,4-[di(3-methylidencampher-10-sulfonsäure)] handelt.

27. Verwendung nach Anspruch 25 oder 26, **dadurch gekennzeichnet, dass** die amphiphile zwitterionische oder kationische Verbindung so wie in einem der Ansprüche 2 bis 6 definiert ist.

## Claims

1. Cosmetic composition for topical use, in particular for photoprotecting the skin and/or the hair, in the form of a dispersion comprising two immiscible phases stabilized with at least one anionic surfactant chosen from the salts of fatty acids and of monovalent and polyvalent metals, of ammonium or of organic bases, a compound for screening out ultraviolet radiation, consisting of a hydrophilic screening agent comprising at least one partially or totally neutralized sulphonic acid function, which can be adsorbed at the interface of the said immiscible phases, having the formula: 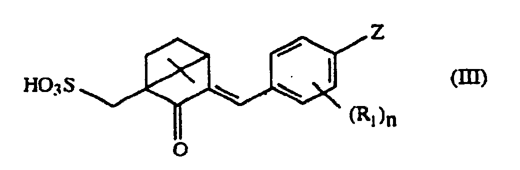 in which:
- Z denotes a group of formula: 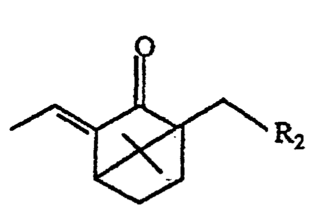
- R₂ denoting -H or -SO₃H;
- n denotes 0 or an integer greater than or equal to 1 and less than or equal to 4;
- R₁ represents one or more identical or different, linear or branched alkyl or alkoxy radicals containing from 1 to 4 carbon atoms,
the two methylidenecamphor radicals on the phenyl ring being in a meta or para position relative to each other,
as well as a metal oxide nanopigmeht chosen from titanium oxide, zinc oxide, iron oxide, zirconium oxide and cerium oxide, and mixtures thereof, coated with hydrophobic hydrocarbon-based coating agents, with an average primary particle size ranging from 5 to 100 nm, this composition being **characterized in that** it also contains a cationic or zwitterionic amphiphilic compound which leads, with the anionic surfactant, to the formation of a compound capable of lowering the water/liquid paraffin interface tension at 40°C by more than 14 mN.m⁻¹ for an anionic surfactant concentration of 0.1 mmol/100 g, by more than 26 mN.m⁻¹ for an anionic surfactant concentration of 0.5 mmol/100 g and by more than 33 mN.m⁻¹ for an anionic surfactant concentration of 1 mmol/100 g.

2. Composition-according to Claim 1, **characterized in that** the cationic or zwitterionic amphiphilic compound has the formula: 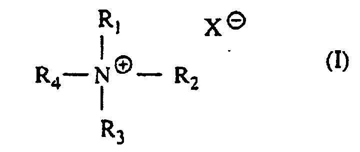 in which:
- R₁ to R₄, which may be identical or different, represent a linear or branched C₁-C₃₀ alkyl, hydroxyalkyl, alkylcarboxylate, alkylsulphonate, hydroxyalkylsulphonate, cycloalkyl, alkenyl, aryl or arylalkyl radical which can be interrupted with one or more hetero atoms such as oxygen or sulphur or with an -NH- or amide -CONH- group, or R₁ and R₂ and/or R₃ and R₄ form, together with the nitrogen atom to which they are attached, a five- or six-membered saturated or unsaturated heterocycle which is unsubstituted or substituted with C₁-C₃₀ alkyl, hydroxyalkyl, alkenyl, aryl or arylalkyl radicals, at least one of the radicals R₁ to R₄ or substituents on the heterocycle, and preferably at least two of these radicals or substituents, comprising at least 6 carbon atoms;
- X⁻ represents an inorganic or organic ion chosen from halide, sulphate, bisulphate, methosulphate, paratoluenesulphonate and saccharinate ions, preferably chloride or bromide; it being understood that when at least .one of the radicals R₁ to R₄ represents an alkylcarboxylate, alkylsulphonate or hydroxyalkylsulphonate radical, the ion X⁻ does not exist.

3. Composition according to Claim 1, **characterized in that** the cationic or zwitterionic amphiphilic compound has the formula: 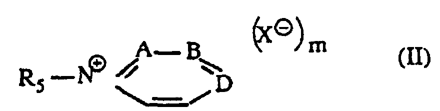 in which:
- R₅ represents a linear or branched C₁-C₃₀ alkyl, hydroxyalkyl, alkylcarboxylate, cycloalkyl, alkenyl, aryl or arylalkyl radical which can be interrupted with one or more hetero atoms such as oxygen or sulphur or with an -NH- or amide -CONH- group;
- A, B or D represents CH, N, N⁺R₆, R₆ representing a hydrogen atom, a substituted or unsubstituted, linear or branched, saturated or unsaturated C₁-C₃₀ alkyl radical which can be interrupted with one or more hetero atoms, it being understood that only one of the symbols A, B or D represents N or N⁺R₆;
with the proviso that at least one of the radicals R₅ or R₆ comprises at least 6 carbon atoms;
- X⁻ represents an inorganic or organic ion chosen from halide, sulphate, bisulphate, methosulphate, paratoluenesulphonate and saccharinate ions, preferably chloride or bromide;
- m = 0, 1 or 2.

4. Composition according to Claim 2 or 3, **characterized in that** the radicals R₁ to R₆ comprising at least 6 carbon atoms are C₈-C₂₂ radicals.

5. Composition according to Claim 2 or 4, **characterized in that** the cationic or zwitterionic amphiphilic compound of formula (I) is chosen from betaines such as decyl betaine, lauryl betaine, lauramidopropyl betaine, myristyl betaine, myristamidopropyl betaine, coco-betaine, cocoamidoethyl betaine, cocoamidopropyl betaine, cetyl betaine, palmamidopropyl betaine, palmitamidopropyl betaine, ricinoleamidopropyl betaine, stearamidopropyl betaine, stearyl betaine, oleyl betaine, oleamidopropyl betaine, or behenyl betaine, sultaines such as lauryl sultaine, lauryl hydroxysultaine, coco-sultaine, coco-hydroxysultaine, cocoamidopropyl hydroxysultaine or oleamidopropyl hydroxysultaine, alkyltrimethylammonium salts such as dodecyltrimethylammonium bromide or chloride, cocotrimethylammonium chloride, cetyltrimethylammonium chloride, bromide, methosulphate or tosylate, (hydrogenated) trimethylammonium tallow chloride, stearyltrimethylammonium chloride, octyldodecyltrimethylammonium chloride, behenyltrimethylammonium chloride or methosulphate or benzalkonium chloride, bromide or saccharinate, cetalkonium chloride, cetearalkonium bromide, lauralkonium chloride or bromide, stearalkonium chloride, olealkonium chloride, behenalkonium chloride and cocoylbenzylhydroxyethylimidazolinium chloride.

6. Composition according to Claim 3 or 4, **characterized in that** the cationic or zwitterionic amphiphilic compound of formula (II) is chosen from cetylpyridinium chloride and laurylpyridinium chloride.

7. Composition according to any one of the preceding claims, **characterized in that** the cationic or zwitterionic amphiphilic compound is present in concentrations ranging from 0.1 to 15% by weight of active material and preferably from 0.1 to 10% by weight of active material, relative to the total weight of the composition.

8. Composition according to any one of the preceding claims, **characterized in that** the compound for screening out ultraviolet radiation, of formula (III), is benzene-1,4-bis(3-methylidene-10-camphorsulphonic acid).

9. Composition according to any one of the preceding claims, **characterized in that** the sulphonic acid function of the compound for screening out ultraviolet radiation, of formula (III), is neutralised with a monovalent or multivalent metal hydroxide, aqueous ammonia or an organic base.

10. Composition according to any one of the preceding claims, **characterized in that** the screening compound of formula (III) is present in concentrations ranging from 0.1 to 10% by weight of active material relative to the total weight of the composition, more particularly from 0.2 to 8% by weight of active material and preferably from 0.5 to 5% by weight of active material.

11. Composition according to any one of the preceding claims, **characterized in that** the anionic surfactant is present in concentrations ranging from 0.5 to 10% by weight of active material relative to the total weight of the composition, and more particularly from 0.5 to 5% by weight of active material.

12. Composition according to any one of the preceding claims, **characterized in that** the metal oxide nanopigment is a titanium oxide nanopigment.

13. Composition according to any one of the preceding claims, **characterized in that** the hydrophobic hydrocarbon-based coating agent comprises a fatty acid or a salt of a fatty acid and of a monovalent or multivalent metal, of ammonium or of an organic base.

14. Composition according to any one of the preceding claims, **characterized in that** the metal oxide nanopigment has an average primary particle size ranging from 10 to 50 nm.

15. Composition according to any one of the preceding claims, **characterized in that** the metal oxide nanopigment is present in a proportion of from 0.1 to 15% by weight relative to the total weight of the composition, and preferably in a proportion of from 0.5 to 10% by weight.

16. Composition according to any one of the preceding claims, **characterized in that** it also contains at least one nonionic surfactant chosen from compounds containing an ester bond, such as fatty acid esters of glycols, fatty acid esters of glycerol, fatty acid esters of polyglycerols, fatty acid esters of tetritol, pentitol and hexitol, fatty acid esters of polyethylene glycols, fatty acid esters of sucrose, triglyceride esters of sucrose, fatty acid esters of sorbitan and polyoxyethylenated sorbitan esters or polysorbates, compounds containing an ether bond, such as polyoxyethylene glycol alkylphenyl ethers and polyoxyethylene glycol fatty alkyl ethers, and compounds containing an amide bond, such as polyoxyethylenated alkylamides and alkylene oxide copolymers.

17. Composition according to Claim 16, **characterized in that** the nonionic surfactants are present in concentrations ranging from 0.5 to 5% by weight of active material relative to the total weight of the composition, and more particularly from 1 to 3% by weight of active material.

18. Composition according to any one of the preceding claims, **characterized in that** it also contains one or more additional hydrophilic or lipophilic UV-B and/or UV-A sunscreens other than the screening agents defined in any one of the preceding claims.

19. Composition according to Claim 18, **characterized in that** the additional sunscreens are chosen from cinnamates, salicylates, benzylidenecamphor derivatives, triazine derivatives, benzophenone derivatives, dibenzoylmethane derivatives, β,β'-diphenyl acrylate derivatives, p-aminobenzoic acid derivatives, benzimidazole derivatives, bis(benzotriazolylphenol) derivatives, screening polymers and screening silicones, and compounds comprising at least two benzazolyl groups.

20. Composition according to any one of the preceding claims, **characterized in that** it also comprises at - least one cosmetic adjuvant chosen from fatty substances, organic solvents, ionic or nonionic thickeners and/or gelling agents, softeners, antioxidants, opacifiers, stabilizers, emollients, hydroxy acids, vitamins, hydrophilic or lipophilic active agents such as ceramides, reducing agents, silicones, free-radical scavengers, antifoaming agents, hydrating agents, fragrances, preserving agents, fillers, sequestering agents, polymers, propellants, acidifying or basifying agents and dyes.

21. Composition according - to any one of the preceding claims, **characterized in that** it constitutes a composition for protecting the human epidermis or an antisun composition and is in the form of a dispersion, an emulsion such as a cream, milk or cream-gel, a solid tube, a foam or a spray.

22. Composition according to any one of Claims 1 to 20, **characterized in that** it constitutes a make-up composition for the eyelashes, the eyebrows or the skin and is in solid, liquid or pasty form, such as an emulsion, a nonionic vesicle dispersion, a powder or a solid tube.

23. Composition according to any one of Claims 1 to 20, which is used for protecting the hair against ultraviolet rays, **characterized in that** it is in the form of a shampoo, an emulsion, a nonionic vesicle dispersion, a foam or a spray.

24. Non-therapeutic process for treating the skin and/or the hair to protect them against the effects of UV rays with wavelengths of between 280 and 400 nm, **characterized in that** it consists in applying an effective amount of a photoprotective cosmetic composition as defined in any one of the preceding claims.

25. Use, for increasing the protection factor of a photoprotective cosmetic composition according to any one of Claims 1 to 23, of a cationic or zwitterionic amphiphilic compound which leads, with the anionic surfactant, to the formation of a compound capable of lowering the water/liquid paraffin interface tension at 40°C by more than 14 mN.m⁻¹ for. an anionic surfactant concentration of 0.1 mmol/100 g, by more than 26 mN.m⁻¹ for an anionic surfactant concentration of 0.5 mmol/100 g and by more than 33 mN.m⁻¹ for an anionic surfactant concentration of 1 mmol/100 g.

26. Use according to Claim 25, **characterized in that** the compound for screening out ultraviolet radiation, of formula (III), is benzene-1,4-bis-(3-methylidene-10-camphorsulphonic acid).

27. Use according to Claim 25 or 26, **characterized in that** the cationic or zwitterionic amphiphilic compound is as defined in any one of Claims 2 to 6.
